# EUROPEAN PATENT SPECIFICATION

(11) **EP 2 084 132 B1**
(45) Date of publication and mention of the grant of the patent: **08.08.2018**
(21) Application number: 06795893.4
(22) Date of filing: 03.09.2006
(51) Int. Cl.: C07D 213/74, C07D 213/80, C07C 237/34, C07C 327/30, A61K 31/245, A61K 31/44, A61P 29/00

(54) **POSITIVELY CHARGED WATER-SOLUBLE PRODRUGS OF N-ARYLANTHRANILIC ACIDS WITH VERY FAST SKIN PENETRATION RATE**
POSITIV GELADENE WASSERLÖSLICHE PRODRUGS VON N-ARYLANTHRANILSÄUREN MIT SEHR SCHNELLER HAUTPENETRATIONSGESCHWINDIGKEIT
PROMÉDICAMENTS HYDROSOLUBLES POSITIVEMENT CHARGÉS D'ACIDES N-ARYLANTHRANILIQUES À VITESSE DE PÉNÉTRATION CUTANÉE TRÈS ÉLEVÉE

(43) Date of publication of application: 05.08.2009
(60) Divisional application: 13153470.3
(73) Proprietor: Techfields Biochem Co. Ltd, Shanghai N/A 200444 (CN); Yu, Chongxi, Kensington, MD 20895 (US)
(72) Inventor: YU, Chongxi, MD 20895 (US); XU, Lina, Shanghai 200444 (CN)
(74) Representative: Finnegan Europe LLP
(86) International application number: PCT/IB2006/053090
(87) International publication number: WO 2008/029199

(56) References cited:
- EP-A2- 0 289 262
- GB-A- 984 471
- GB-A- 1 000 208
- GB-A- 1 165 300
- GB-A- 1 187 259
- JP-A- 57 183 738
- US-A- 2 671 805
- US-A- 3 420 871
- VENUTI M.C. ET AL.: 'Synthesis and biological Evaluation of omega-(N,N,N-Trialkylammonium)alkyl Esters and Thioesters of Carboxylic Acid Nonsteroidal Antiinflammatory Agents' PHARMACEUTICAL RESEARCH vol. 6, no. 10, 1989, pages 867 - 873, XP001098334
- NEBIOGLU D. ET AL.: 'Synthesis and in vitro anti-inflammatory activities of some new diaryl amine derivatives as prodrug of diclofenac' JOURNAL OF FACULTY OF PHARMACY OF GAZI UNIVERSITY vol. 10, no. 1, 1993, pages 69 - 81, XP008110133
- ROTH H.J. ET AL.: 'Synthese polymergebundener Antiphlogistika' ARCHIV. DER PHARMAZIE vol. 321, no. 5, 1988, pages 273 - 276, XP008109571
- DATABASE CA [Online] SALIMBENI A. ET AL.: 'New esters of N-arylanthranilic acids', XP008110610 Retrieved from STN Database accession no. (83:274 & FARMACO, EDIZIONE SCIENTIFICA vol. 30, no. 4, 1975, pages 277 - 286
- URBANSKA H. ET AL.: 'Synthesis and pharmacological properties of aminoalkyl esters derived from nicotinic acid' ACTA POLONICAE PHARMACEUTICA vol. 36, no. 6, 1979, pages 657 - 665, XP008082803
- GIDOH M ET AL: "Studies on the derivatives showing local anesthetic actions of several acidic antiinflammatory drugs, aiming at the possibility of treatment for leprous neuritis", NIHON RAIGAKKAI ZASSHI - JAPANESE JOURNAL OF LEPROSY, NIHON RAI GAKUKAI, AOMORI, JP, vol. 52, no. 3, 1 July 1983 (1983-07-01), pages 156-164, XP002698719, ISSN: 0386-3980

## Description

### Technical Field

The present invention relates to the preparations of positively charged and watersoluble pro-drugs of arylanthranilic acids and related compounds and their medicinal use in treating any nonsteroidal anti-inflammatory drugs (NSAIAs)-treatable conditions in humans or animals. More specifically, the present invention is to overcome the side effects that are associated with the use of NSAIAs. These pro-drugs can be administered orally or transdermally.

### Background Art

2-[(2,3-Dimethylphenyl)amino]benzoic acid (mefenamic acid), 2-[(2,6-dichloro-3-methylphenyl)amino]benzoic acid (meclofenamic acid), 2-[[(3-trifluoromethyl)phenyl]amino]benzoic acid (flufenamic acid), 2-[[3-(trifluoromethyl)phenyl]amino]-3-pyridinecarboxylic acid (niflumic acid), 2-[[2-methyl-3-(trifluoromethyl)phenyl]amino]-3-pyridinecarboxylic acid (flunixin) and related compounds are members of arylanthranilic acid group of nonsteroidal anti-inflammatory drugs. They are used for the relief of signs and symptoms of rheumatoid arthritis and osteoarthritis and for the treatment of dysmenorrhea. They are also used for the treatment of acute gouty arthritis and ankylosing spondylitis.

Unfortunately, a number of side effects are associated with the use of mefenamic acid, meclofenamic acid, flufenamic acid, niflumic acid, flunixin, and related compounds, most notably GI disturbances such as dyspepsia, gastroduodenal bleeding, gastric ulcerations, and gastritis. Fishman (Fishman; Robert, U.S. Pat. No. 7,052,715) indicated that an additional problem associated with oral medications, is that the concentration levels which must be achieved in the bloodstream must be significant in order to effectively treat distal areas of pain or inflammation. These levels are often much higher than would be necessary if it were possible to accurately target the particular site of pain or injury. Fishman and many others (Van Engelen et al. U.S. Pat. No. 6,416,772; Macrides et al. U.S. Pat. No. 6,346,278; Kirby et al. U.S. Pat. No. 6,444,234, Roentsch, et al., U.S. Pat. No. 5,654,337, Park, et al., U.S. Pat. No. 6,190,690, Pearson et al. U.S. Pat. No. 6,528,040, and Botknecht et al. U.S. Pat. No. 5,885,597) have tried to develop a delivery system for transdermal application by formulation. It is very difficult, however, to deliver therapeutically effective plasma levels of these kind drugs into the host by formulation, due to the slow skin penetration rate. Susan Milosovich, et al. designed and prepared testosteronyl-4-dimethylaminobutyrate.HCl (TSBH), which has a lipophilic portion and a tertiary amine groups that exists in the protonated form at physiological pH. They found that the prodrug (TSBH) diffuses through human skin ∼60 times faster than does the drug (TS) itself [Susan Milosovich, et al., J. Pharm. Sci., 82, 227(1993)].

### Disclosure of Invention

### Technical Problem

Mefenamic acid, meclofenamic acid, flufenamic acid, niflumic acid, flunixin, and related compounds have been used medicinally for many years. They are used for the relief of signs and symptoms of rheumatoid arthritis and osteoarthritis, for the treatment of dysmenorrhea.

Unfortunately, a number of side effects are associated with the use of mefenamic acid, meclofenamic acid, flufenamic acid, niflumic acid, flunixin, and related compounds, most notably GI disturbances such as dyspepsia, gastroduodenal bleeding, gastric ulcerations, and gastritis. They are not soluble in aqueous solution and gastric juice. They stay in the GI tract for a long time and thus, may cause gastric mucosal cell damage.

### Technical Solution

The present invention provides a compound having a general formula of Structure 1 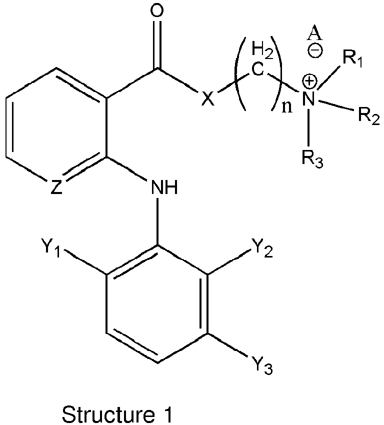 wherein
a) R₁ represents H; R₂ represents H, an alkyl, alkyloxy, alkenyl or alkynyl residue having 1 to 12 carbon atoms, or an aryl residue; R₃ represents H, an alkyl, alkyloxy, alkenyl or alkynyl residue having 1 to 12 carbon atoms, or an aryl residue; X represents O, S, or NH; A⁻ represents Cl⁻, Br⁻, F⁻, I⁻, AcO⁻, citrate, or a negative ion; Y₁ represents H, Cl, F, CH₃, C₂H₅, or CF₃; Y₂ represents H, Cl, F, CH₃, C₂H₅, or CF₃; Y₃ represents H, Cl, F, CH₃, C₂H₅, or CF₃; Z represents CH; and n= 0, 1, 2, 3, 4, 5, 6, 7, 8, 9, or 10 or a composition comprising at least one compound having the general formula of Structure 1 as an active ingredient for use in a method for treating a non-steroidal anti-inflammatory agent (NSAIA)-treatable condition in a subject, wherein the subject is human or animal, the compound or composition is administered transdermally, and the non-steroidal anti-inflammatory agent (NSAIA)-treatable condition is selected from the group consisting of pain, pain from a toothache, headache, arthritis pain, inflammatory pain, fever, cancer, dysmenorrhea, radiation-induced vomiting, diabetic neuropathy, acute migraine, hemophilic arthropathy, bone loss, sunburn, eye inflammatory diseases, ocular pain after corneal surgery, glaucoma, ear inflammatory and painful conditions (otitis) in a subject, rheumatoid arthritis, osteoarthritis, psoriasis, acne, sunburn, and other skin disorders; or
b) R₁ represents H; R₂ represents H, an alkyl, alkyloxy, alkenyl or alkynyl residue having 1 to 12 carbon atoms, or an aryl residue; R₃ represents H, an alkyl, alkyloxy, alkenyl or alkynyl residue having 1 to 12 carbon atoms, or an aryl residue; X represents S or NH; A⁻ represents Cl⁻, Br⁻, F⁻, I⁻, AcO⁻, citrate, or a negative ion; Y₁ represents H, Cl, F, CH₃, C₂H₅, or CF₃; Y₂ represents H, Cl, F, CH₃, C₂H₅, or CF₃; Y₃ represents Cl, F, CH₃, C₂H₅, or CF₃; Z represents CH or N; and n= 0, 1, 2, 3, 4, 5, 6, 7, 8, 9, or 10 or a composition comprising at least one compound having the general formula of Structure 1 as an active ingredient for use in a method for treating a non-steroidal anti-inflammatory agent (NSAIA)-treatable condition in a subject, wherein the subject is human or animal, the compound or composition is administered transdermally, and the non-steroidal anti-inflammatory agent (NSAIA)-treatable condition is selected from the group consisting of pain, pain from a toothache, headache, arthritis pain, inflammatory pain, fever, cancer, dysmenorrhea, radiation-induced vomiting, diabetic neuropathy, acute migraine, hemophilic arthropathy, bone loss, asthma, sunburn, eye inflammatory diseases, ocular pain after corneal surgery, glaucoma, ear inflammatory and painful conditions (otitis) in a subject, rheumatoid arthritis, osteoarthritis, psoriasis, acne, sunburn, and other skin disorders.

This invention relates to the preparation of novel positively charged pro-drugs of arylanthranilic acids and related compounds and their use medicinally. The pro-drugs of arylanthranilic acids have the general formula (1) 'Structure 1'.

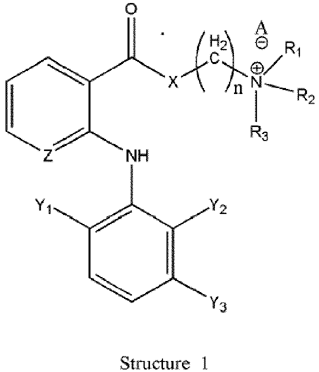

In structure 1, R₁ represents H, one of any alkyl, alkyloxyl, alkenyl or alkynyl residues having 1 to 12 carbon atoms, or aryl residues; R₂ represents H, one of any alkyl, alkyloxy, alkenyl or alkynyl residues having 1 to 12 carbon atoms, or aryl residues; R₃ represents H, one of any alkyl, alkyloxy, alkenyl or alkynyl residues having 1 to 12 carbon atoms, or aryl residues; X represents O, S, or NH; A⁻ represents Cl⁻, Br⁻, F⁻, I⁻, AcO⁻, citrate, or any negative ions; Y₁ represents H, Cl, F, CH₃, C₂H₅, or CF₃ ; Y₂ represents H, Cl, F, CH₃, C₂H₅, or CF₃; Y₃ represents H, Cl, F, CH₃, C₂H₅, or CF₃; Z represents CH or N; and n=0, 1, 2, 3, 4, 5, 6, 7, 8, 9, 10 ....... All R groups may include C, H, O, S, or N atoms and may have single, double, and treble bonds. Any CH₂ groups may be replaced with O, S, or NH.

Drug absorption, whether from the gastrointestinal tract or other sites, requires the passage of the drug in a molecular form across the barrier membrane. The drug must first dissolve, and if the drug possesses the desirable biopharmaceutical properties, it will pass from a region of high concentration to a region of low concentration across the membrane into the blood or general circulation. All biological membranes contain lipids as major constituents. The molecules that play the dominant roles in membrane formation all have phosphate-containing highly polar head groups, and, in most cases, two highly hydrophobic hydrocarbon tails. Membranes are bilayers, with the hydrophilic head groups facing outward into the aqueous regions on either side. Very hydrophilic drugs cannot pass the hydrophobic layer of membrane and very hydrophobic drugs will stay in the hydrophobic layer as part of the membrane due to their similarities and cannot enter the cytosol on the inside efficiently.

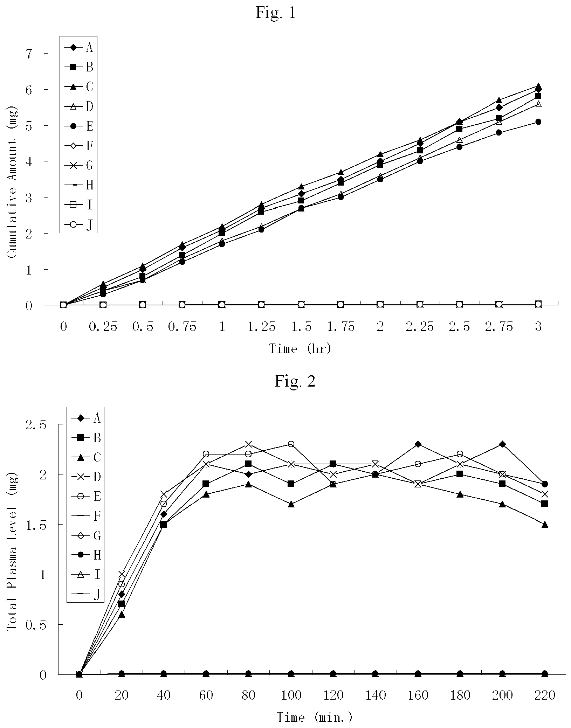

The goal of this invention is to avoid the side effects of mefenamic acid, meclofenamic acid, flufenamic acid, niflumic acid, flunixin, and related compounds by increasing the their solubility in gastric juice and their penetration rate through the membrane and skin barrier which will make it administrable transdermally (topical application). These novel pro-drugs have two structural features in common: they have a lipophilic portion and a primary, secondary, or tertiary amine group that exists in the protonated form (hydrophilic part) at physiological pH. Such a hydrophilic-lipophilic balance is required for efficient passage through the membrane barrier [Susan Milosovich, et al., J. Pharm. Sci., 82, 227(1993)]. The positively charged amino groups largely increase the solubility of the drugs. The solubility of diethylaminoethyl 2-[(2,3-dimethylphenyl)amino]benzoate.AcOH, diethylaminoethyl 2-[(2,6-dichloro-3-methylphenyl)amino]benzoate.AcOH, diethylaminoethyl 2-[[(3-(trifluoromethyl)phenyl)amino]benzoate.AcOH, diethylaminoethyl 2-[[3-(trifluoromethyl)phenyl]amino]-3-pyridinecarboxylate.AcOH, diethylaminoethyl 2-[[2-methyl-3-(trifluoromethyl)phenyl]amino]-3-pyridinecarboxylate.AcOH, mefenamic acid, meclofenamic acid, flufenamic acid, niflumic acid, and flunixin in water are >400 mg, >400 mg, >400 mg, >450 mg, >450 mg, <0.1 mg, <0.1 mg, <0.1 mg, <0.1 mg, <0.1 mg/ml, In many instances, the lowest or rate-limiting step in the sequence is the dissolution of the drug. Mefenamic acid, meclofenamic acid, flufenamic acid, niflumic acid, flunixin, and related compounds have a very low solubility in gastric juice. They stay in the GI tract for a long time and thus, may cause gastric mucosal cell damage. When these new pro-drugs are administered orally in a dosage form such as a tablet, capsule, solution, or suspension, they will dissolve in the gastric juice immediately. The positive charge on the amino groups of these pro-drugs will bond to the negative charge on the phosphate head group of membrane. Thus, the local concentration of the outside of the membrane will be very high and will facilitate the passage of these pro-drugs from a region of high concentration to a region of low concentration. When these pro-drugs enter the membrane, the hydrophilic part will push the pro-drug into the cytosol, a semi-liquid concentrated aqueous solution or suspension. Due to the short stay in GI tract, the pro-drugs will not cause gastric mucosal cell damage. The pH of the stomach is 1-3, so the negative charge on the phosphate head group of the membrane of the gastric mucosa is bonded with proton (H ⁺). The positive charges of these prodrugs cannot bond to phosphate head group of the gastric mucosa. These prodrugs will be free both of primary insult (direct acid damage) and secondary insult (prostaglandin inhibition) to the stomach. The penetration rates of diethylaminoethyl 2-[(2,3-dimethylphenyl)amino]benzoate.AcOH, diethylaminoethyl 2-[(2,6-dichloro-3-methylphenyl)amino]benzoate.AcOH, diethylaminoethyl 2-[[(3-(trifluoromethyl)phenyl)amino]benzoate.AcOH, diethylaminoethyl 2-[[3-(trifluoromethyl)phenyl]amino]-3-pyridinecarboxylate.AcOH, diethylaminoethyl 2-[[2-methyl-3-(trifluoromethyl)phenyl]amino]-3-pyridinecarboxylate.AcOH, mefenamic acid, meclofenamic acid, flufenamic acid, niflumic acid, flunixin, and related compounds through human skin were measured in vitro by using modified Franz cells, which were isolated from human skin tissue (360-400 µm thick) of the anterior and posterior thigh areas. The receiving fluid consisted of 10 ml of 2% bovine serum albumin in normal saline and was stirred at 600 rpm. The cumulative amounts of these prodrugs and their parent drugs penetrating the skin versus time were determined by a specific high-performance liquid chromatography method. The results using a donor consisting of either a 30% solution of diethylaminoethyl 2-[(2,3-dimethylphenyl)amino]benzoate.AcOH, diethylaminoethyl 2-[(2,6-dichloro-3-methylphenyl)amino]benzoate.AcOH, diethylaminoethyl 2-[[(3-(trifluoromethyl)phenyl)amino]benzoate.AcOH, diethylaminoethyl 2-[[3-(trifluoromethyl)phenyl]amino]-3-pyridinecarboxylate.AcOH, and diethylaminoethyl 2-[[2-methyl-3-(trifluoromethyl)phenyl]amino]-3-pyridinecarboxylate.AcOH or a 30% suspension of mefenamic acid, meclofenamic acid, flufenamic acid, niflumic acid, and flunixin in 2mL of pH 7.4 phosphate buffer (0.2M) are shown in Figure 1. Apparent flux values of 2.1 mg, 2.0 mg, 2.2 mg, 1.8 mg, 1.7 mg, 0.01 mg, 0.01 mg, 0.01 mg, 0.01 mg, and 0.01 mg/cm²/h were calculated for diethylaminoethyl 2-[(2,3-dimethylphenyl)amino]benzoate.AcOH, diethylaminoethyl 2-[(2,6-dichloro-3-methylphenyl)amino]benzoate.AcOH, diethylaminoethyl 2-[[(3-(trifluoromethyl)phenyl)amino]benzoate.AcOH, diethylaminoethyl 2-[[3-(trifluoromethyl)phenyl]amino]-3-pyridinecarboxylate.AcOH, and diethylaminoethyl 2-[[2-methyl-3-(trifluoromethyl)phenyl]amino]-3-pyridinecarboxylate.AcOH, mefenamic acid, meclofenamic acid, flufenamic acid, niflumic acid, and flunixin diffuses through human skin. The results suggest that the pro-drugs, diethylaminoethyl 2-[(2,3-dimethylphenyl)amino]benzoate.AcOH, diethylaminoethyl 2-[(2,6-dichloro-3-methylphenyl)amino]benzoate.AcOH, diethylaminoethyl 2-[[(3-(trifluoromethyl)phenyl)amino]benzoate.AcOH, diethylaminoethyl 2-[[3-(trifluoromethyl)phenyl]amino]-3-pyridinecarboxylate.AcOH, or diethylaminoethyl 2-[[2-methyl-3-(trifluoromethyl)phenyl]amino]-3-pyridinecarboxylate.AcOH diffuses through human skin ∼200 times faster than does mefenamic acid, meclofenamic acid, flufenamic acid, niflumic acid, or flunixin. The results suggest that the positive charge on the dialkyaminoethyl group has a very important role in the passage of the drug across the membrane and skin barrier. Other prodrugs of the general 'Structure 1' have very high penetration rates and are very close to that of diethylaminoethyl 2-[(2,3-dimethylphenyl)-amino]benzoate.AcOH.

The in vivo rates of penetration of diethylaminoethyl 2-[(2,3-dimethylphenyl)amino]benzoate.AcOH, diethylaminoethyl 2-[(2,6-dichloro-3-methylphenyl)amino]benzoate.AcOH, diethylaminoethyl 2-[[(3-(trifluoromethyl)phenyl)amino]benzoate.AcOH, diethylaminoethyl 2-[[3-(trifluoromethyl)phenyl]amino]-3-pyridinecarboxylate.AcOH, and diethylaminoethyl 2-[[2-methyl-3-(trifluoromethyl)phenyl]amino]-3-pyridinecarboxylate.AcOH, mefenamic acid, meclofenamic acid, flufenamic acid, niflumic acid, and flunixin through the skin of intact hairless mice were compared. The donor consisted of a 20% solution of these compounds in 1 mL of isopropanol applied to a 10 cm² on the backs of the hairless mice. Plasma levels of mefenamic acid, meclofenamic acid, flufenamic acid, niflumic acid, and flunixin were determined by a specific high-performance liquid chromatography method. The results (Figure 2) show that the peak levels of diethylaminoethyl 2-[(2,3-dimethylphenyl)amino] benzoate.AcOH, diethylaminoethyl 2- [(2,6-dichloro-3-methylphenyl)amino] benzoate.AcOH, diethylaminoethyl 2-[[(3-(trifluoromethyl)phenyl)amino] benzoate.AcOH, diethylaminoethyl 2-[[3-(trifluoromethyl)phenyl]amino] - 3-pyridinecarboxylate.AcOH, and diethylaminoethyl 2-[[2-methyl-3-(trifluoromethyl)phenyl]amino]-3-pyridinecarboxylate.AcOH were reached in -50 minutes after application of the donor systems. It takes 2-4 hours for mefenamic acid, meclofenamic acid, flufenamic acid, niflumic acid, flunixin, and related compounds to reach their peak plasma level when they are taken orally. The peak plasma levels were ∼0.01 mg/ml for mefenamic acid, meclofenamic acid, flufenamic acid, niflumic acid, and flunixin and ∼2 mg/ml for diethylaminoethyl 2-[(2,3-dimethylphenyl)amino]benzoate.AcOH, diethylaminoethyl 2-[(2,6-dichloro-3-methylphenyl)amino]benzoate.AcOH, diethylaminoethyl 2-[[(3-(trifluoromethyl)phenyl)amino]benzoate.AcOH, diethylaminoethyl 2-[[3-(trifluoromethyl)phenyl]amino]-3-pyridinecarboxylate.AcOH, and diethylaminoethyl 2-[[2-methyl-3-(trifluoromethyl)phenyl]amino]-3-pyridinecarboxylate.AcOH (approximately 200 times difference). ∼2 mg/ml of mefenamic acid, meclofenamic acid, flufenamic acid, niflumic acid, and flunixin in plasma is more than ∼ 50 times higher than plasma level for effective analgesia and effective antiinflammatory activity. This is a very exciting result. It will be very easy and fast to deliver therapeutically effective plasma level of mefenamic acid, meclofenamic acid, flufenamic acid, niflumic acid, and flunixin into the host by administration of these prodrugs transdermally. These results suggest that the pro-drugs can be administered not only orally, but also transdermally for any kind of medical treatments. The in vivo rates of penetration of other Pro-drugs of the general 'Structure 1' are close to that of diethylaminoethyl diethylaminoethyl 2-[(2,3-dimethylphenyl)amino]benzoate.AcOH.

To check the gastroduodenal bleeding caused by drugs, rats were orally administered with 50 mg/kg of diethylaminoethyl 2-[(2,3-dimethylphenyl)amino] benzoate.AcOH, diethylaminoethyl 2- [(2,6-dichloro-3-methylphenyl)amino] benzoate.AcOH, diethylaminoethyl 2-[[(3-(trifluoromethyl)phenyl)amino] benzoate.AcOH, diethylaminoethyl 2-[[3-(trifluoromethyl)phenyl]amino] - 3-pyridinecarboxylate.AcOH, diethylaminoethyl 2-[[2-methyl-3-(trifluoromethyl)phenyl]amino]-3-pyridinecarboxylate.AcOH, mefenamic acid, meclofenamic acid, flufenamic acid, niflumic acid, and flunixin per day for 21 days. We found an average of 2-4 mg of fecal blood per gram of feces in the mefenamic acid, meclofenamic acid, flufenamic acid, niflumic acid, and flunixin groups and none in diethylaminoethyl 2-[(2,3-dimethylphenyl)amino]benzoate.AcOH, diethylaminoethyl 2-[(2,6-dichloro-3-methylphenyl)amino]benzoate.AcOH, diethylaminoethyl 2-[[(3-(trifluoromethyl)phenyl)amino]benzoate.AcOH, diethylaminoethyl 2-[[3-(trifluoromethyl)phenyl]amino]-3-pyridinecarboxylate.AcOH, diethylaminoethyl 2-[[2-methyl-3-(trifluoromethyl)phenyl]amino]-3-pyridinecarboxylate.AcOH.

The acute toxicity of the prodrugs was investigated. The LD₅₀ orally in mice are: 0.9 g/kg, 1.0 g/kg, 0.8g/kg , 0.75 g/kg, 1.1 g/kg , for diethylaminoethyl 2-[(2,3-dimethylphenyl)amino]benzoate.AcOH, diethylaminoethyl 2-[(2,6-dichloro-3-methylphenyl)amino]benzoate.AcOH, diethylaminoethyl 2-[[(3-(trifluoromethyl)phenyl)amino]benzoate.AcOH, diethylaminoethyl 2-[[3-(trifluoromethyl)phenyl]amino]-3-pyridinecarboxylate.AcOH, diethylaminoethyl 2-[[2-methyl-3-(trifluoromethyl)phenyl]amino]-3-pyridinecarboxylate.AcOH. The results show that the prodrugs are less toxic than their parent drugs (mefenamic acid, 600 mg/kg, flufenamic acid, 715 mg/kg, and niflumic acid, 650 mg/kg).

Mefenamic acid, meclofenamic acid, flufenamic acid, niflumic acid, and flunixin demonstrated anti-inflammatory, analgesic, antipyretic, and antirheumatic activity. A good prodrug should go back to the parent drug in plasma. Diethylaminoethyl ester group of these prodrugs can be rapidly cleaved by the enzymes in human plasma in vitro and more than 90% of the pro-drugs are changed back to their parent drugs. Due to the pro-drugs having a much better absorption rate, the prodrugs will have more strength than their parent drugs at the same dosage. The analgetic, antipyretic, and anti-inflammatory activities of these prodrugs were tested using mefenamic acid, meclofenamic acid, flufenamic acid, niflumic acid, and flunixin as a comparison.

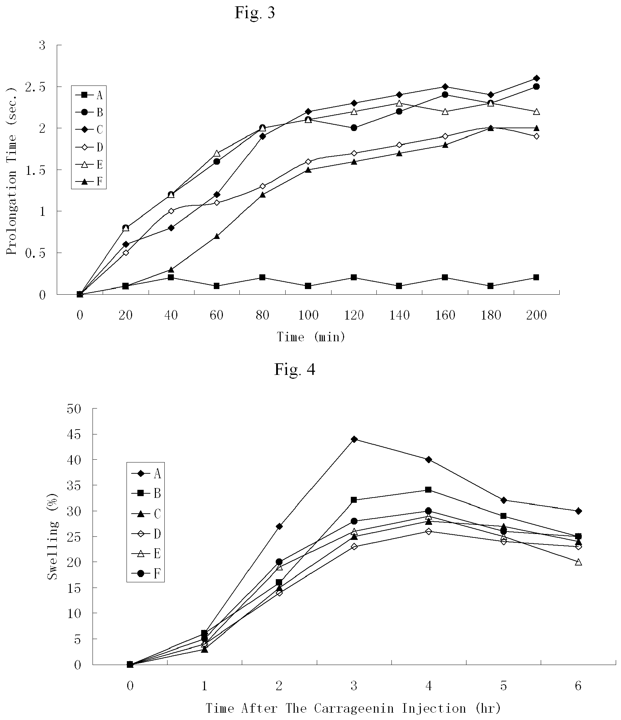

Analgetic activity: The prolongation time of the pain threshold of a mouse tail was determined in accordance with the D'Amour-Smith Method (J. Pharmacol. Exp. Ther., 72, 74(1941)). After 50mg/kg of these prodrugs were administered transdermally, the tails of mice were exposed to heat and the prolongation time of pain threshold was determined. The results obtained are shown in Figure 3. Diethylaminoethyl 2-[(2,3-dimethylphenyl)amino]benzoate.AcOH, diethylaminoethyl 2-[(2,6-dichloro-3-methylphenyl)amino]benzoate.AcOH, diethylaminoethyl 2-[[(3-(trifluoromethyl)phenyl)amino]benzoate.AcOH, diethylaminoethyl 2-[[3-(trifluoromethyl)phenyl]amino]-3-pyridinecarboxylate.AcOH, diethylaminoethyl 2-[[2-methyl-3-(trifluoromethyl)phenyl]amino]-3-pyridinecarboxylate.AcOH have shown analgesic activity nicely.

The number of writhings that occurred when mice were administered an acetic acid solution intraperitoneally were counted, and the rate of inhibition based on the control group was calculated. diethylaminoethyl 2-[(2,3-dimethylphenyl)amino] benzoate.AcOH (100 mg/kg, B), diethylaminoethyl 2-[(2,6-dichloro-3-methylphenyl)amino]benzoate.AcOH (100 mg/kg, C), diethylaminoethyl 2-[[(3-(trifluoromethyl)phenyl)amino]benzoate.AcOH (100 mg/kg, D), diethylaminoethyl 2-[[3-(trifluoromethyl)phenyl]amino]-3-pyridinecarboxylate.AcOH (100 mg/kg, E), diethylaminoethyl 2-[[2-methyl-3-(trifluoromethyl)phenyl]amino] - 3-pyridinecarboxylate.AcOH (100 mg/kg, F) were administered transdermally the mice 60 minutes before the acetic acid solution was administered. The group A is the control group. The results are shown in Table 1.

**Table 1. The rate of writhings inhibition by prodrugs of arylanthranilic acids**

| Group | Dose (mg/kg) | No. of Writhings | % |
|---|---|---|---|
| A | 0 | 35.0 | - |
| B | 100 | 15.6 | 55 |
| C | 100 | 14.2 | 59 |
| D | 100 | 16.1 | 54 |
| E | 100 | 15.2 | 57 |
| F | 100 | 15.7 | 55 |

The results show that the prodrugs demonstrate exceptional analgetic activity. Other compounds of the general 'Structure 1' show similar analgetic activity.

Antipyretic activity: Rats received a sterilized E. coli suspension as a pyrogen. The control group is group A. 2 hours later, diethylaminoethyl 2-[(2,3-dimethylphenyl)amino]benzoate.AcOH (100 mg/kg, B), diethylaminoethyl 2-[(2,6-dichloro-3-methylphenyl)amino]benzoate.AcOH (100 mg/kg, C), diethylaminoethyl 2-[[(3-(trifluoromethyl)phenyl)amino]benzoate.AcOH (100 mg/kg, D), diethylaminoethyl 2-[[3-(trifluoromethyl)phenyl]amino]-3-pyridinecarboxylate.AcOH (100 mg/kg, E), diethylaminoethyl 2-[[2-methyl-3-(trifluoromethyl)phenyl]amino] - 3-pyridinecarboxylate.AcOH (100 mg/kg, F) were administered transdermally. The body temperature of rats was taken at 90 min. intervals before and after the administration of the test compounds. The results are shown in Table 2.

**Table 2. Antipyretic Activity of prodrugs of arylanthranilic acids.**

| Compound | t=0 min. | t=90 min. | t=180 min. | t=270 min. |
|---|---|---|---|---|
| A (Control group) | 37.34±0.05 | 37.36±0.07 | 37.37±0.05 | 37.44±0.08 |
| B (100mg/kg) | 37.35±0.06 | 36.71±0.05 | 36.60±0.08 | 36.59±0.07 |
| C (100mg/kg) | 37.28±0.06 | 36.68±0.05 | 36.62±0.08 | 36.58±0.07 |
| D (100mg/kg) | 37.27±0.06 | 36.76±0.05 | 36.65±0.08 | 36.49±0.07 |
| E (100mg/kg) | 37.25±0.07 | 36.82±0.06 | 36.70±0.05 | 36.50±0.08 |
| F (100mg/kg) | 37.23±0.06 | 36.69±0.06 | 36.52±0.08 | 36.40±0.07 |

The results shown that the prodrugs demonstrated strong antipyretic activity at 100 mg/kg dose. Other compounds of the general 'Structure 1' show similar antipyretic activity.

Anti-inflammatory activity: diethylaminoethyl 2-[(2,3-dimethylphenyl)amino] benzoate.AcOH (100 mg/kg, B), diethylaminoethyl 2-[(2,6-dichloro-3-methylphenyl)amino]benzoate.AcOH (100 mg/kg, C), diethylaminoethyl 2-[[(3-(trifluoromethyl)phenyl)amino]benzoate.AcOH (100 mg/kg, D), diethylaminoethyl 2-[[3-(trifluoromethyl)phenyl]amino]-3-pyridinecarboxylate.AcOH (100 mg/kg, E), diethylaminoethyl 2-[[2-methyl-3-(trifluoromethyl)phenyl]amino] - 3-pyridinecarboxylate.AcOH (100 mg/kg, F) were administered transdermally. Group A is the controlled group. 60 minutes later, a carrageenin solution was administered subcutaneously to the foot pads of the rats. The volume of the hind paw was measured at every hour after the administration of the carrageenin, and the rate of increase in the volume of the paw was calculated and designated as the rate of swelling (%). The results obtained are shown in Figure 4. The results show that these prodrugs by transdermal administration demonstrated good anti-inflammatory activity. Other compounds of the general 'Structure 1' show similar anti-inflammatory activity.

It is also known that a high oral dose of some of NSAIAs shows an anti-reactive-antiasthmatic activity by inhibition of the cyclooxygenase activity. Due to their very high membrane penetration rate, these prodrugs can be used in treating asthma by spraying into the mouth or nose of the host.

These prodrugs can also be used to treat psoriasis, acne, sunburn or other skin disorders due to their anti-inflammatory properties and very high skin penetration rate.

Recent attention has been drawn to the role of cyclooxygenase (COX)-2 in the pathogenesis of cancer and it has been considered as an attractive target for strategies in cancer patients. These prodrugs can also be used to treat skin cancer, lung cancer, breast cancer,colon cancer, oral cancer, genital cancer, and other cancers by administering to the cancer area a very high effective amount directly.

The present invention relates to pharmaceutical preparations comprising of prodrugs of the general 'Structure 1' in addition to customary auxiliaries and excipients, e.g. in the form of tablets, capsules or solutions for administration orally and in the form of solutions, lotion, ointment, emulsion or gel for transdermal administration. The new active compounds of the general 'Structure 1' can be combined with vitamins such as A, B, C or E or beta-carotene, or other pharmaceuticals, such as folic acid, etc., for treating any NSAIAs-treatable conditions in humans or animals.

Transdermal therapeutic application systems of compounds of the general 'Structure 1' or a composition comprising of at least one compound of the general 'Structure 1', as an active ingredient, can be used for treating any NSAIAs-treatable conditions in humans or animals. These systems can be a bandage or a patch comprising of one active substance-containing matrix layer and an impermeable backing layer. The most preferable system is an active substance reservoir, which has a permeable bottom facing the skin. By controlling the rate of release, this system enables NSAIAs to reach constantly optimal therapeutic blood levels to increase effectiveness and reduce the side effects of NSAIAs. These systems can be worn on the wrist, ankle, arm, leg, or any part of body.

The compounds of the general formula (1) 'Structure 1' indicated above can be prepared from mefenamic acid, meclofenamic acid, flufenamic acid, niflumic acid, flunixin, and related compounds, by reaction with compounds of the general formula (2) 'Structure 2' by using coupling reagents, such as N,N'-Dicyclohexylcarbodiimide, N, N'-Diisopropylcarbodiimide, O-(Benzotriazol-1-yl)-N,N,N',N'-tetramethyluronium tetrafluoroborate, O-(Benzotriazol-1-yl)-N,N,N',N'-tetramethyluronium hexafluorophosphate, Benzotriazol-1-yl-oxy-tris(dimethylamino)phosphonium hexafluorophosphate, et al.

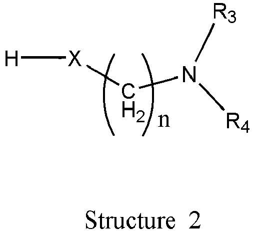

In structure 2, R₃ represents H, one of any alkyl, alkyloxy, alkenyl, or alkynyl residues having 1 to 12 carbon atoms, or aryl residues; R₄ represents H, one of any alkyl, alkyloxy, alkenyl, or alkynyl residues having 1 to 12 carbon atoms, or aryl residues; X represents O, S or NH; and n=0, 1, 2, 3, 4, 5, 6, 7, 8, 9, 10........

When X represents O, the compounds of the general formula (1) 'Structure 1' indicated above can be prepared from metal salts or organic base salts of mefenamic acid, meclofenamic acid, flufenamic acid, niflumic acid, flunixin, and related compounds, by reaction with compounds of the general formula (3) "Structure".

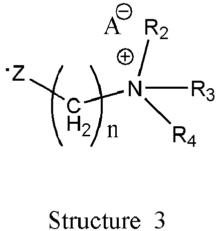

In structure 3, R₂ represents H, one of any alkyl, alkyloxy, alkenyl, or alkynyl residues having 1 to 12 carbon atoms, or aryl residues; R₃ represents H, one of any alkyl, alkyloxy, alkenyl, or alkynyl residues having 1 to 12 carbon atoms, or aryl residues; R₄ represents H, one of any alkyl, alkyloxy, alkenyl, or alkynyl residues having 1 to 12 carbon atoms, or aryl residues; Z represents halogen, or p-toluenesulphonyl, A⁻represents Cl⁻, Br⁻, F⁻, I⁻, AcO⁻, citrate, or any negative ions; and n=0, 1, 2, 3, 4, 5, 6, 7, 8, 9, 10.....

When X represents O, the compounds of the general formula (1) 'Structure 1' indicated above can be prepared from immobilized base salts of mefenamic acid, meclofenamic acid, flufenamic acid, niflumic acid, flunixin, and related compounds of the general formula (4) 'Structure 4',

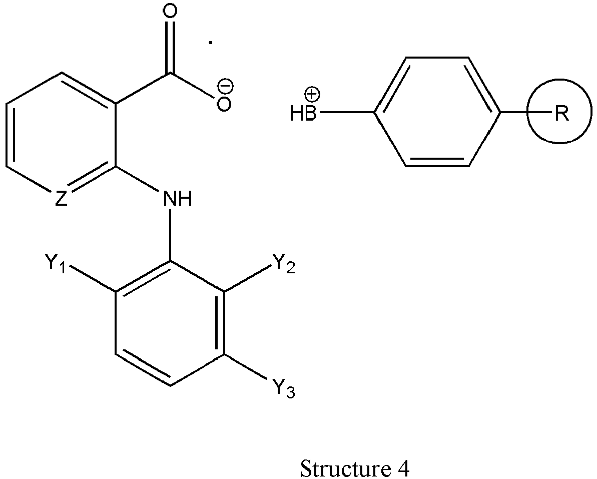

In structure 4, R represents cross-linked resin; Y₁ represents H, Cl, F, CH₃, C₂H₅, or CF ₃; Y₂ represents H, Cl, F, CH , CH , or CF₃; Y represents H, Cl, F, CH₃, C₂H₅, or CF₃; Z represents CH or N; B represents any base groups, such as pyridine, piperidine, triethylamine, or other base groups, by reaction with compounds of the general formula (3) 'Structure 3' .

### Advantageous Effects

These pro-drugs of mefenamic acid, meclofenamic acid, flufenamic acid, niflumic acid, flunixin, and related compounds have a lipophilic portion and a hydrophilic portion (the amine groups that exist in the protonated form at physiological pH). The positively charged amino groups of these pro-drugs have two major advantages. First, it largely increases the solubility of the drugs; when these new pro-drugs are administered orally in a dosage form such as a tablet, capsule, solution, or suspension, they will dissolve in gastric juice immediately. Second, the positive charge on the amino group of these pro-drugs will bond to the negative charge on the phosphate head group of membrane. Thus, the local concentration outside of the membrane will be very high and will facilitate the passage of these pro-drugs from a region of high concentration to a region of low concentration. When these pro-drugs enter the membrane, the hydrophilic part will push the pro-drugs into the cytosol, a semi-liquid concentrated aqueous solution or suspension. Due to the short stay in the GI tract, the pro-drugs will not cause gastric mucosal cell damage. Experiment results show that more than 90% of the pro-drugs were changed back to the parent drugs. The pro-drugs have a much better absorption rate, and thus the pro-drugs will have better strength than mefenamic acid, meclofenamic acid, flufenamic acid, niflumic acid, flunixin, and related compounds at the same dosage. The experiment results suggest that the pro-drugs, diethylaminoethyl 2-[(2,3-dimethylphenyl)amino]benzoate.AcOH, diethylaminoethyl 2-[(2,6-dichloro-3-methylphenyl)amino]benzoate.AcOH, diethylaminoethyl 2-[[(3-(trifluoromethyl)phenyl)amino]benzoate.AcOH, diethylaminoethyl 2-[[3-(trifluoromethyl)phenyl]amino]-3-pyridinecarboxylate.AcOH, or diethylaminoethyl 2-[[2-methyl-3-(trifluoromethyl)phenyl]amino]-3-pyridineca rboxylate.AcOH diffuses through human skin ∼200 times faster than does mefenamic acid, meclofenamic acid, flufenamic acid, niflumic acid, or flunixin. It takes 2-4 hours for mefenamic acid, meclofenamic acid, flufenamic acid, niflumic acid, flunixin, and related compounds to reach the peak plasma level when they are taken orally, but these prodrugs only took about ∼50 minutes to reach the peak plasma level. The most exciting result is that the pro-drugs can be administered not only orally, but also transdermally for any type of medical treatment and should avoid most of the side effects of mefenamic acid, meclofenamic acid, flufenamic acid, niflumic acid, or flunixin, and related compounds, most notably GI disturbances such as dyspepsia, gastroduodenal bleeding, gastric ulcerations, gastritis, and renal toxicity. Another great benefit of transdermal administration of these pro-drugs is that administering medication, especially to children, will be much easier.

### Description of Drawings

Figure 1: Cumulative amounts of diethylaminoethyl 2-[(2,3-dimethylphenyl)amino ]benzoate.AcOH (A, 30% solution), diethylaminoethyl 2-[(2,6-dichloro-3-methylphenyl)amino]benzoate.AcOH (B, 30% solution), diethylaminoethyl 2-[[(3-(trifluoromethyl)phenyl)amino]benzoate.AcOH (C, 30% solution), diethylaminoethyl 2-[[3-(trifluoromethyl)phenyl]amino]-3-pyridinecarboxylate.AcOH (D, 30% solution), diethylaminoethyl 2-[[2-methyl-3-(trifluoromethyl)phenyl]amino] - 3-pyridinecarboxylate.AcOH (E, 30% solution), mefenamic acid (F, 30% suspension), meclofenamic acid (G, 30% suspension), flufenamic acid (H, 30% suspension), niflumic acid (I, 30% suspension), flunixin (J, 30% suspension), crossing isolated human skin tissue in Franz cells (n=5). In each case, the vehicle was pH 7.4 phosphate buffer (0.2 M).
Figure 2: Total plasma levels of mefenamic acid, meclofenamic acid, flufenamic acid, niflumic acid, and flunixin after topical application of 1 ml of a 20% solution of diethylaminoethyl 2-[(2,3-dimethylphenyl)amino]benzoate.AcOH (A), diethylaminoethyl 2-[(2,6-dichloro-3-methylphenyl)amino]benzoate.AcOH (B), diethylaminoethyl 2-[[(3-(trifluoromethyl)phenyl)amino]benzoate.AcOH (C), diethylaminoethyl 2-[[3-(trifluoromethyl)phenyl]amino]-3-pyridinecarboxylate.AcOH (D), diethylaminoethyl 2-[[2-methyl-3-(trifluoromethyl)phenyl]amino]-3-pyridinecarboxylate.AcOH (E), mefenamic acid (F), meclofenamic acid (G), flufenamic acid (H), niflumic acid (I), and flunixin (J) in isopropanol to the backs of hairless mice (n=5).
Figure 3: The prolongation time of the pain threshold of mice tails after 50mg/kg of diethylaminoethyl 2-[(2,3-dimethylphenyl)amino]benzoate.AcOH, diethylaminoethyl 2-[(2,6-dichloro-3-methylphenyl)amino]benzoate.AcOH, diethylaminoethyl 2-[[(3-(trifluoromethyl)phenyl)amino]benzoate.AcOH, diethylaminoethyl 2-[[3-(trifluoromethyl)phenyl]amino]-3-pyridinecarboxylate.AcOH, diethylaminoethyl 2-[[2-methyl-3-(trifluoromethyl)phenyl]amino]-3-pyridinecarboxylate.AcOH were administered transdermally. Group A is the control group.
Figure 4. The rate of swelling (%) after a carrageenin injection. 1 hour before the carrageenin injection, diethylaminoethyl 2-[(2,3-dimethylphenyl)amino] benzoate.AcOH (100 mg/kg, B), diethylaminoethyl 2-[(2,6-dichloro-3-methylphenyl)amino]benzoate.AcOH (100 mg/kg, C), diethylaminoethyl 2-[[(3-(trifluoromethyl)phenyl)amino]benzoate.AcOH (100 mg/kg, D), diethylaminoethyl 2-[[3-(trifluoromethyl)phenyl]amino]-3-pyridinecarboxylate.AcOH (100 mg/kg, E), diethylaminoethyl 2-[[2-methyl-3-(trifluoromethyl)phenyl]amino] - 3-pyridinecarboxylate.AcOH (100 mg/kg, F) were administered transdermally. Group A is the control group.

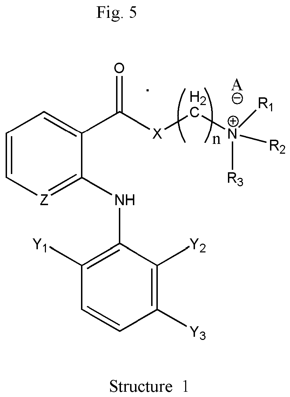

Figure 5. In structure 1, R₁ represents H, one of any alkyl, alkyloxyl, alkenyl or alkynyl residues having 1 to 12 carbon atoms, or aryl residues; R₂ represents H, one of any alkyl, alkyloxy, alkenyl or alkynyl residues having 1 to 12 carbon atoms, or aryl residues; R₃ represents H, one of any alkyl, alkyloxy, alkenyl or alkynyl residues having 1 to 12 carbon atoms, or aryl residues; X represents O, S, or NH; A⁻ represents Cl⁻, Br⁻, F⁻, I⁻, AcO⁻, citrate, or any negative ions; Y represents H, Cl, F, CH₃, C₂H₅, or CF₃; Y₂ represents H, Cl, F, CH₃, C₂H₅, or CF₃; Y₃ represents H, Cl, F, CH₃, C₂H₅, or CF₃; Z represents CH or N; and n=0, 1, 2, 3, 4, 5, 6, 7, 8, 9, 10 ......; All R groups may include C, H, O, S, or N atoms and may have single, double, and treble bonds. Any CH ₂ groups may be replaced with O, S, or NH

### Best Mode

### Preparation of N-diethylaminoethyl 2-[(2,3-dimethylphenyl)amino] benzoamide.AcOH.

24.1 g (0.1 mol) of 2-[(2,3-dimethylphenyl)amino]benzoic acid was dissolved in 100 ml of acetonitrile. 32.1 g of O-(Benzotriazol-1-yl)-N,N,N',N'-tetramethyluronium tetrafluoroborate and 30 ml of triethylamine were added into the reaction mixture. 11.6 g of dimethylaminoethylamine was added into the reaction mixture. The mixture was stirred for 3 hours at RT. The solvents were evaporated off. 250 ml of ethyl acetate was added into the reaction mixture and the mixture was washed with water (3 x 100 ml). The organic solution was dried over anhydrous sodium sulfate. Sodium sulfate was removed by filtration. 6 g of acetic acid was added into the reaction mixture with stirring. Hexane (200 ml) was added. The solid product was collected by filtration. After drying, it yielded 37 g of the desired product (92.5%). Hygroscopic product; Solubility in water: 400 mg/ml; Elementary analysis: C₂₃H₃₃N₃O₄; MW: 399.53. Calculated % C: 69.14; H: 8.33; N: 10.52; O: 12.01; Found % C: 69.11; H: 8.35; N: 10.51; O: 12.03. ¹H-NMR (400 MHz, D₂O): δ: 1.41 (t, 6H), 2.10 (s, 3H), 2.30 (s, 3H), 2.31 (s, 3H), 3.22 (m, 4H), 3.54 (m, 2H), 3.60 (m, 2H), 6.15 (m, 1H), 6.30 (m, 1H), 6.57 (m, 1H), 6.72 (m, 1H), 7.20 (m, 2H), 7.70 (m, 1H), 7.80 (b, 1H).

### Mode for Invention

### Preparation of N-diethylaminoethyl 2-[(2,6-dichloro-3-methylphenyl)amino] benzoamide.AcOH

29.6 g (0.1 mol) of 2-[(2,6-dichloro-3-methylphenyl)amino]benzoic acid was dissolved in 300 ml of chloroform. 20.6 g of N, N'-Dicyclohexylcarbodiimide was added into the reaction mixture. 11.7 g of diethylaminoethylamine was added into the reaction mixture. The mixture was stirred for 3 hours at RT. The solid is removed by filtration. The chloroform solution was washed with 5% NaHCO₃ (2 x 100 ml) and water (3 x 100 ml). The organic solution was dried over anhydrous sodium sulfate. Sodium sulfate was removed by filtration. 6 g of acetic acid was added into the reaction mixture with stirring. Hexane (200 ml) was added. The solid product was collected by filtration. After drying, it yielded 39 g of the desired product (85.8%). Hygroscopic product; Solubility in water: 400 mg/ml; Elementary analysis: C₂₂H₂₉Cl₂N₃O ₃; MW: 454.39. Calculated % C: 58.15; H: 6.43; Cl: 15.60; N: 9.25, O: 10.56; Found % C: 58.10; H: 6.46; Cl: 15.62; N: 9.22, O: 10.60. ¹H-NMR (400 MHz, D₂O): δ: 1.43 (t, 6H), 2.11 (s, 3H), 2.28 (s, 3H), 3.23 (m, 4H), 3.49 (m, 2H), 3.63 (m, 2H), 6.30 (d, 1H), 6.57 (m, 1H), 6.72 (d, 1H), 6.80 (m, 1H), 7.20 (m, 1H), 7.68 (m, 1 H), 7.70 (b, 1H).

### Preparation of S-dimethylaminoethyl 2-[[(3-trifluoromethyl)phenyl]amino] benzoate.AcOH.

28.1 g (0.1 mol) of 2-[[(3-trifluoromethyl)phenyl]amino]benzoic acid was dissolved in 300 ml of chloroform. N, N'-Dicyclohexylcarbodiimide was added into the reaction mixture. 11.7 g of dimethylaminoethyl mercaptan was added into the reaction mixture. The mixture was stirred for 3 hours at RT. The solid is removed by filtration. The chloroform solution was washed with 5% NaHCO₃ (2 x 100 ml) and water (3 x 100 ml). The organic solution was dried over anhydrous sodium sulfate. Sodium sulfate was removed by filtration. 6 g of acetic acid was added into the reaction mixture with stirring. Hexane (200 ml) was added. The solid product was collected by filtration. After drying, it yielded 39 g of the desired product (88.5%). Hygroscopic product; Solubility in water: 400 mg/ml; Elementary analysis: C₂₁H₂₆F₃N₃O₃S; MW: 456.52. Calculated % C: 57.88; H: 5.96; F: 12.48; N: 6.14, O: 10.51; S: 7.02; Found % C: 57.84; H: 5.99; F: 12.45; N: 6.15, O: 10.56, S: 7.01. ¹H-NMR (400 MHz, D₂O): δ: 1.44 (t, 6H), 2.11 (s, 3H), 3.23 (m, 4H), 3.30 (m, 2H), 3.90 (m, 2H), 6.46 (m, 1H), 6.65 (m, 2H), 6.77 (m, 2H), 6.90 (m, 1H), 7.30 (m, 1H), 7.78 (m, 1 H).

### Preparation of diethylaminoethyl 2-[[3-(trifluoromethyl)phenyl]amino] - 3-pyridinecarboxylate.AcOH

28.2 g (0.1 mol) of 2-[[3-(trifluoromethyl)phenyl]amino]-3-pyridinecarboxylic acid was dissolved in 200 ml of 10% NaHCO . 100 ml of acetone and 43 g (0.15mol) of di-ethylaminoethyl bromide.HBr was added into the mixture and the mixture was stirred for 5 hours at RT. The mixture is extracted with ethyl acetate (2 x 300 ml). The ethyl acetate solution is dried over anhydrous sodium sulfate. 6 g of acetic acid is added into the solution. The solution was concentrated in vacuo to 100 ml. Then 300 ml of hexane was added into the solution. The solid product was collected by filtration and washed with hexane (3 x 100 ml). After drying, it yielded 38 g of the desired product (86.1%). Hygroscopic product; Solubility in water: 400 mg/ml; Elementary analysis: C₂₁H₂₆F₃N₃ 04; MW: 441.44. Calculated % C: 57.14; H: 5.94; F: 12.91, N: 9.52; O: 14.50; Found % C: 57.11; H: 5.97; F: 12.92; N: 9.50; O: 14.50. ¹H-NMR (400 MHz, D₂O): δ: 1.44 (t, 6H), 2.11 (s, 3H), 3.23 (m, 4H), 3.70 (m, 2H), 4.60 (m, 2H), 6.46 (m, 1H), 6.65 (s, 1H), 6.77 (m, 1H), 6.83 (m, 1H), 6.90 (m, 1H), 8.00 (m, 1H), 8.38 (m, 1 H).

### Preparation of diethylaminoethyl 2-[[2-methyl-3-(trifluoromethyl)phenyl] amino]-3-pyridinecarboxylate.AcOH

60 g of Polymer-bound triethylamine (3 mol/g, 100-200 mesh) was suspended in 180 ml of chloroform. 29.6 g (0.1 mol) of 2-[[2-methyl-3-(trifluoromethyl)phenyl] amino]-3-pyridinecarboxylic acid was added into the mixture with stirring. 43 g (0.15mol) of diethylaminoethyl bromide.HBr was added into the mixture and the mixture was stirred for 5 hours at RT. The polymer was removed by filtration and washed with tetrahydrofuran (3 x 50 ml). 8.2 g (0.1 mol) of sodium acetate was added into the reaction mixture with stirring. The mixture was stirred for 2 h. The solid was removed by filtration and washed with chloroform (3 x 50 ml). The solution was concentrated in vacuo to 100 ml. Then 300 ml of hexane was added into the solution. The solid product was collected by filtration and washed with hexane (3 x 100 ml). After drying, it yielded 40 g of the desired product (87.8%). Hygroscopic product; Solubility in water: 400 mg/ml; Elementary analysis: C₂₂H₂₈F₃N₃O₄; MW: 455.47. Calculated % C: 58.01; H: 6.20; F: 12.51, N: 9.23; O: 14.05; Found % C: 57.98; H: 6.23; F: 12.50; N: 9.21; O: 14.08. ¹H-NMR (400 MHz, D₂O): δ: 1.45 (t, 6H), 2.11 (s, 3H), 2.35 (s, 3H), 3.23 (m, 4H), 3.70 (m, 2H), 4.60 (m, 2H), 6.36 (m, 1H), 6.65 (m, 1H), 6.77 (m, 1H), 6.83 (m, 1H), 8.00 (m, 1H), 8.38 (m, 1 H).

### Industrial Applicability

The pro-drugs of the general formula (1) 'Structure 1' are superior to mefenamic acid, meclofenamic acid, flufenamic acid, niflumic acid, flunixin, and related compounds. They can be used for treating any mefenamic acid, meclofenamic acid, flufenamic acid, niflumic acid, flunixin, and related compounds-treatable conditions in humans or animals. They may be used for the relief of signs and symptoms of rheumatoid arthritis and osteoarthritis, the reduction of fever, and the treatment of dysmenorrhea. They may be also prescribed for diabetic neuropathy and acute migraine headache. Due to their very high membrane penetration rate, these pro-drugs can be used in treating asthma by inhalation to a host. They can be used to treat psoriasis, acne, sunburn or other skin disorders due to their anti-inflammatory properties. They can also be used for treating skin cancer, lung cancer, breast cancer,colon cancer, oral cancer, genital cancer, and other cancers by administering to the cancer area a very high effective amount directly.

**Sequence List Text**

## Claims

1. A compound having a general formula of Structure 1 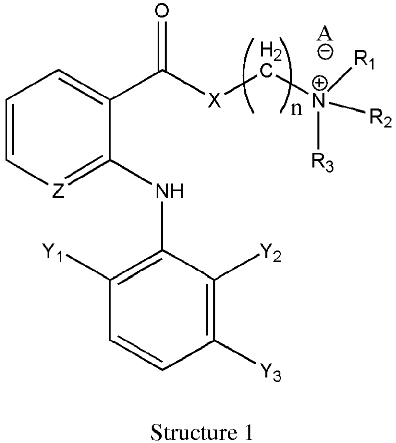 wherein
a) R₁ represents H; R₂ represents H, an alkyl, alkyloxy, alkenyl or alkynyl residue having 1 to 12 carbon atoms, or an aryl residue; R₃ represents H, an alkyl, alkyloxy, alkenyl or alkynyl residue having 1 to 12 carbon atoms, or an aryl residue; X represents O, S, or NH; A⁻ represents Cl⁻, Br⁻, F⁻, I⁻, AcO⁻, citrate, or a negative ion; Y₁ represents H, Cl, F, CH₃, C₂H₅, or CF₃; Y₂ represents H, Cl, F, CH₃, C₂H₅, or CF₃; Y₃ represents H, Cl, F, CH₃, C₂H₅, or CF₃; Z represents CH; and n= 0, 1, 2, 3, 4, 5, 6, 7, 8, 9, or 10 or a composition comprising at least one compound having the general formula of Structure 1 as an active ingredient for use in a method for treating a non-steroidal anti-inflammatory agent (NSAIA)-treatable condition in a subject, wherein the subject is human or animal, the compound or composition is administered transdermally, and the non-steroidal anti-inflammatory agent (NSAIA)-treatable condition is selected from the group consisting of pain, pain from a toothache, headache, arthritis pain, inflammatory pain, fever, cancer, dysmenorrhea, radiation-induced vomiting, diabetic neuropathy, acute migraine, hemophilic arthropathy, bone loss, sunburn, eye inflammatory diseases, ocular pain after corneal surgery, glaucoma, ear inflammatory and painful conditions (otitis) in a subject, rheumatoid arthritis, osteoarthritis psoriasis, acne, sunburn, and other skin disorders; or
b) R₁ represents H; R₂ represents H, an alkyl, alkyloxy, alkenyl or alkynyl residue having 1 to 12 carbon atoms, or an aryl residue; R₃ represents H, an alkyl, alkyloxy, alkenyl or alkynyl residue having 1 to 12 carbon atoms, or an aryl residue; X represents S or NH; A⁻ represents Cl⁻, Br⁻, F⁻, I⁻, AcO⁻, citrate, or a negative ion; Y₁ represents H, Cl, F, CH₃, C₂H₅, or CF₃; Y₂ represents H, Cl, F, CH₃, C₂H₅, or CF₃; Y₃ represents Cl, F, CH₃, C₂H₅, or CF₃; Z represents CH or N; and n= 0, 1, 2, 3, 4, 5, 6, 7, 8, 9, or 10 or a composition comprising at least one compound having the general formula of Structure 1 as an active ingredient for use in a method for treating a non-steroidal anti-inflammatory agent (NSAIA)-treatable condition in a subject, wherein the subject is human or animal, the compound or composition is administered transdermally, and the non-steroidal anti-inflammatory agent (NSAIA)-treatable condition is selected from the group consisting of pain, pain from a toothache, headache, arthritis pain, inflammatory pain, fever, cancer, dysmenorrhea, radiation-induced vomiting, diabetic neuropathy, acute migraine, hemophilic arthropathy, bone loss, asthma, sunburn, eye inflammatory diseases, ocular pain after corneal surgery, glaucoma, ear inflammatory and painful conditions (otitis) in a subject, rheumatoid arthritis, osteoarthritis psoriasis, acne, sunburn, and other skin disorders.

2. The compound or composition for use according to claim 1, wherein the compound is selected from the group consisting of:
diethylaminoethyl 2-[(2,3-dimethylphenyl)amino]benzoate.AcOH, diethylaminoethyl 2-[(2,6-dichloro-3-methylphenyl)amino]benzoate.AcOH, and diethylaminoethyl 2-[[(3-(trifluoromethyl)phenyl)amino]benzoate. AcOH.

3. The compound or composition for use according to any one of claims 1-2, wherein the compound or composition is administered transdermally to a part of body of the subject in the form of a solution, spray, lotion, ointment, emulsion or gel to provide a therapeutically effective plasma level of the compound.

4. The compound or composition for use according to any one of claims 1-3 for topically treating a pain in the subject by administering the compound or composition transdermally to the inflamed area of the subject.

5. The compound or composition for use according to claim 4, wherein the pain is headache, toothache, and muscle pain, or inflammatory pain.

6. The compound or composition for use according to claim 1, wherein the compound or composition is administered transdermally in the form of a solution, spray, lotion, ointment, emulsion, or gel for treating psoriasis, acne, sunburn, skin disorders, or cancer.

7. The compound or composition for use according to claim 6, wherein the cancer is skin cancer, lung cancer, breast cancer, colon cancer, oral cancer, or genital cancer.

8. The compound or composition for use according to claim 1, wherein the compound or composition is in the form of a transdermal therapeutic application system, wherein the transdermal therapeutic application system is a bandage or a patch comprising one active substance-containing matrix layer and an impermeable backing layer.

9. The compound or composition for use according to claim 8, wherein the transdermal therapeutic application system is an active substance reservoir, which has a permeable bottom facing the skin of the subject.

## Patentansprüche

1. Verbindung der allgemeinen Formel der Struktur I 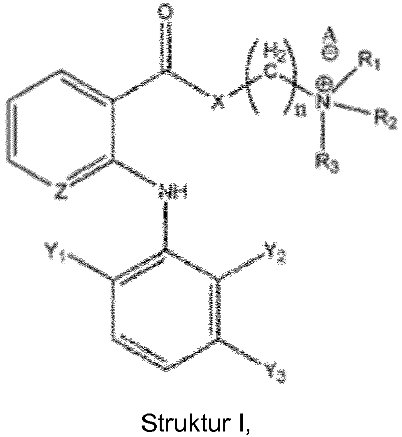 wobei
a) R₁ für H steht; R₂ H, einen Alkyl-, Alkyloxy-, Alkenyl- oder Alkynylrest mit 1 bis 12 Kohlenstoffatomen oder einen Arylrest darstellt; R₃ H, einen Alkyl-, Alkyloxy-, Alkenyl- oder Alkynylrest mit 1 bis 12 Kohlenstoffatomen oder einen Arylrest darstellt; X O, S oder NH darstellt; A⁻ Cl⁻, Br⁻, F⁻, I⁻, AcO⁻, Citrat oder ein negatives Ion darstellt; Y₁ H, Cl, F, CH₃, C₂H₅ oder CF₃ darstellt; Y₂ H, Cl, F, CH₃, C₂H₅ oder CF₃ darstellt; Y₃ H, Cl, F, CH₃, C₂H₅ oder CF₃ darstellt; Z für CH steht; und n = 0, 1, 2, 3, 4, 5, 6, 7, 8, 9 oder 10 ist oder eine Zusammensetzung, die mindestens eine Verbindung, welche die allgemeine Formel der Struktur I aufweist, als Wirkstoff zur Verwendung in einem Verfahren zur Behandlung eines mit einem nicht-steroidalen Entzündungshemmer (NSAIA) behandelbaren Zustands in einem Subjekt umfasst, wobei das Subjekt ein Mensch oder ein Tier ist, wobei die Verbindung oder Zusammensetzung transdermal verabreicht wird, und wobei der mit einem nicht-steroidalen Entzündungshemmer (NSAIA) behandelbare Zustand ausgewählt ist aus der Gruppe bestehend aus Schmerzen, Schmerzen durch Zahnschmerzen, Kopfschmerzen, Arthritisschmerzen, Entzündungsschmerzen, Fieber, Krebs, Dysmenorrhö, strahlungsinduziertem Erbrechen, diabetischer Neuropathie, akuter Migräne, hämophiler Arthropathie, Knochenschwund, Sonnenbrand, Augenentzündungserkrankungen, Augenschmerzen nach Hornhaut-OP, Glaukom, Ohrenentzündungen und Ohrenschmerzen (Otitis) in einem Subjekt, rheumatoider Arthritis, Osteoarthritis, Psoriasis, Akne, Sonnenbrand und andere Hauterkrankungen; oder
b) R₁ für H steht; R₂ H, einen Alkyl-, Alkyloxy-, Alkenyl- oder Alkynylrest mit 1 bis 12 Kohlenstoffatomen oder einen Arylrest darstellt; R₃ H, einen Alkyl-, Alkyloxy-, Alkenyl- oder Alkynylrest mit 1 bis 12 Kohlenstoffatomen oder einen Arylrest darstellt; X S oder NH darstellt; A⁻ Cl⁻, Br⁻, F⁻, I⁻, AcO⁻, Citrat oder ein negatives Ion darstellt; Y₁ H, Cl, F, CH₃, C₂H₅ oder CF₃ darstellt; Y₂ H, Cl, F, CH₃, C₂H₅ oder CF₃ darstellt; Y₃ H, Cl, F, CH₃, C₂H₅ oder CF₃ darstellt; Z für CH oder N steht; und n = 0, 1, 2, 3, 4, 5, 6, 7, 8, 9 oder 10 ist oder eine Zusammensetzung, die mindestens eine Verbindung, welche die allgemeine Formel der Struktur I aufweist, als Wirkstoff zur Verwendung in einem Verfahren zur Behandlung eines mit einem nicht-steroidalen Entzündungshemmer (NSAIA) behandelbaren Zustands in einem Subjekt umfasst, wobei das Subjekt ein Mensch oder ein Tier ist, wobei die Verbindung oder Zusammensetzung transdermal verabreicht wird, und wobei der mit einem nicht-steroidalen Entzündungshemmer (NSAIA) behandelbare Zustand ausgewählt ist aus der Gruppe bestehend aus Schmerzen, Schmerzen durch Zahnschmerzen, Kopfschmerzen, Arthritisschmerzen, Entzündungsschmerzen, Fieber, Krebs, Dysmenorrhö, strahlungsinduziertem Erbrechen, diabetischer Neuropathie, akuter Migräne, hämophiler Arthropathie, Knochenschwund, Asthma, Sonnenbrand, Augenentzündungserkrankungen, Augenschmerzen nach Hornhaut-OP, Glaukom, Ohrenentzündungen und Ohrenschmerzen (Otitis) in einem Subjekt, rheumatoider Arthritis, Osteoarthritis, Psoriasis, Akne, Sonnenbrand und andere Hauterkrankungen.

2. Verbindung oder Zusammensetzung zur Verwendung nach Anspruch 1, wobei die Verbindung ausgewählt ist aus der Gruppe bestehend aus:
Diethylaminoethyl 2-[(2,3-dimethylphenyl)amino]benzoat.AcOH, Diethylaminoethyl 2-[(2,6-dichlor-3-methylphenyl)amino]benzoat.AcOH, und Diethylaminoethyl 2-[[(3-(trifluormethyl)phenyl)amino]benzoat.AcOH.

3. Verbindung oder Zusammensetzung zur Verwendung nach Anspruch 1 oder 2, wobei die Verbindung oder Zusammensetzung transdermal an einen Körperteil des Subjekts verabreicht wird in Form einer Lösung, eines Sprays, einer Lotion, einer Salbe, einer Emulsion oder eines Gels, um einen therapeutisch wirksamen Plasmawert der Verbindung bereitzustellen.

4. Verbindung oder Zusammensetzung zur Verwendung nach einem der Ansprüche 1 bis 3 zur topischen Behandlung eines Schmerzustands in dem Subjekt durch transdermales Verabreichen der Verbindung oder Zusammensetzung an den entzündeten Bereich des Subjekts.

5. Verbindung oder Zusammensetzung zur Verwendung nach Anspruch 4, wobei die Schmerzen Kopfschmerzen, Zahnschmerzen und Muskelschmerzen oder Entzündungsschmerzen sind.

6. Verbindung oder Zusammensetzung zur Verwendung nach Anspruch 1, wobei die Verbindung oder Zusammensetzung transdermal verabreicht wird in Form einer Lösung, eines Sprays, einer Lotion, einer Salbe, einer Emulsion oder eines Gels zur Behandlung von Psoriasis, Akne, Sonnenbrand, Hauterkrankungen oder Krebs.

7. Verbindung oder Zusammensetzung zur Verwendung nach Anspruch 6, wobei der Krebs Hautkrebs, Lungenkrebs, Brustkrebs, Dickdarmkrebs, Mundkrebs oder Krebs im Genitalbereich ist.

8. Verbindung oder Zusammensetzung zur Verwendung nach Anspruch 1, wobei die Verbindung oder Zusammensetzung in Form eines transdermalen therapeutischen Applikationssystems gegeben ist, wobei das transdermale therapeutische Applikationssystem ein Verband oder ein Pflaster ist, das eine Matrixschicht, die eine aktive Substanz enthält, und eine undurchlässige Trägerschicht umfasst.

9. Verbindung oder Zusammensetzung zur Verwendung nach Anspruch 8, wobei das transdermale therapeutische Applikationssystem ein Speicher für eine aktive Substanz ist, mit einer durchlässigen Unterseite, die zu der Haut des Subjekts gerichtet ist.

## Revendications

1. Composé ayant une formule générale de structure 1 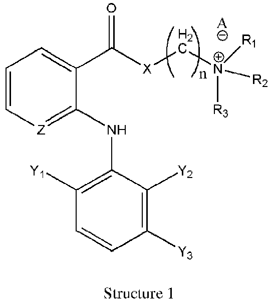 où :
a) R₁ représente H ; R₂ représente H, un résidu alkyle, alkyloxy, alcényle ou alcynyle ayant 1 à 12 atomes de carbone, ou un résidu aryle ; R₃ représente H, un résidu alkyle, alkyloxy, alcényle ou alcynyle ayant 1 à 12 atomes de carbone, ou un résidu aryle ; X représente O, S ou NH ; A⁻ représente Cl⁻, Br⁻, F⁻, I⁻, AcO⁻, citrate, ou un ion négatif; Y₁ représente H, Cl, F, CH₃, C₂H₅, ou CF₃; Y₂ représente H, Cl, F, CH₃, C₂H₅, ou CF₃; Y₃ représente H, Cl, F, CH₃, C₂H₅, ou CF₃ ; Z représente CH ; et n = 0, 1, 2, 3, 4, 5, 6, 7, 8, 9, ou 10 ou une composition comprenant au moins un composé ayant la formule générale de structure 1 comme ingrédient actif à utiliser dans un procédé de traitement d'un état pouvant être traité par un agent anti-inflammatoire non stéroïdien (AINS) chez un sujet, le sujet étant humain ou animal, le composé ou la composition étant administré par voie transdermique, et l'état pouvant être traité par un agent anti-inflammatoire non stéroïdien (AINS) étant choisi dans le groupe constitué par une douleur, mal de dents, mal de tête, douleur arthritique, douleur inflammatoire, fièvre, cancer, dysménorrhée, vomissements radio-induits, neuropathie diabétique, migraine aiguë, arthropathie hémophile, perte osseuse, coups de soleil, maladies inflammatoires oculaires, douleur oculaire après opération de la cornée, glaucome, inflammation de l'oreille et états douloureux (otite) chez un sujet, arthrite rhumatoïde, arthrose, psoriasis, acné, coups de soleil et autres troubles cutanés ; ou
b) R₁ représente H ; R₂ représente H, un résidu alkyle, alkyloxy, alcényle ou alcynyle ayant 1 à 12 atomes de carbone, ou un résidu aryle ; R₃ représente H, un résidu alkyle, alkyloxy, alcényle ou alcynyle ayant 1 à 12 atomes de carbone, ou un résidu aryle ; X représente S ou NH ; A⁻ représente Cl⁻, Br⁻, F⁻, I⁻, AcO⁻, citrate, ou un ion négatif; Y₁ représente H, Cl, F, CH₃, C₂H₅, ou CF₃; Y₂ représente H, Cl, F, CH₃, C₂H₅, ou CF₃; Y₃ représente H, Cl, F, CH₃, C₂H₅, ou CF₃ ; Z représente CH ou N ; et n = 0, 1, 2, 3, 4, 5, 6, 7, 8, 9, ou 10 ou une composition comprenant au moins un composé ayant la formule générale de structure 1 comme ingrédient actif à utiliser dans un procédé de traitement d'un état pouvant être traité par un agent anti-inflammatoire non stéroïdien (AINS) chez un sujet, le sujet étant humain ou animal, le composé ou la composition étant administré par voie transdermique, et l'état pouvant être traité par un agent anti-inflammatoire non stéroïdien (AINS) étant choisi dans le groupe constitué par une douleur, mal de dents, mal de tête, douleur arthritique, douleur inflammatoire, fièvre, cancer, dysménorrhée, vomissements radio-induits, neuropathie diabétique, migraine aiguë, arthropathie hémophile, perte osseuse, asthme, coups de soleil, maladies inflammatoires oculaires, douleur oculaire après opération de la cornée, glaucome, inflammation de l'oreille et états douloureux (otite) chez un sujet, arthrite rhumatoïde, arthrose, psoriasis, acné, coups de soleil et autres troubles cutanés.

2. Composé ou composition destiné à être utilisé selon la revendication 1, le composé étant choisi dans le groupe constitué par :
diéthylaminoéthyl 2-[(2,3-diméthylphényl)amino]benzoate.AcOH, diéthylaminoéthyl 2-[(2,6-dichloro-3-méthylphényl)amino]benzoate.AcOH, et diéthylaminoéthyl 2-[[(3-(trifluorométhyl)phényl)amino]benzoate.AcOH.

3. Composé ou composition destiné à être utilisé selon la revendication 1 ou 2, le composé ou la composition étant administré par voie transdermique à une partie du corps du sujet sous la forme d'une solution, d'une pulvérisation, d'une lotion, d'un onguent, d'une émulsion ou d'un gel pour obtenir un taux plasmatique thérapeutiquement efficace du composé.

4. Composé ou composition à utiliser selon l'une quelconque des revendications 1 à 3 pour le traitement topique d'une douleur chez le sujet en administrant le composé ou la composition par voie transdermique à la zone enflammée du sujet.

5. Composé ou composition à utiliser selon la revendication 4, la douleur étant un mal de tête, un mal de dents et une douleur musculaire, ou une douleur inflammatoire.

6. Composé ou composition à utiliser selon la revendication 1, le composé ou la composition étant administré par voie transdermique sous la forme d'une solution, d'un spray, d'une lotion, d'un onguent, d'une émulsion ou d'un gel pour traiter le psoriasis, l'acné, les coups de soleil, les troubles cutanés ou le cancer.

7. Composé ou composition destiné à être utilisé selon la revendication 6, le cancer étant un cancer de la peau, un cancer du poumon, un cancer du sein, un cancer du côlon, un cancer de la bouche ou un cancer génital.

8. Composé ou composition à utiliser selon la revendication 1, le composé ou la composition étant sous la forme d'un système d'application thérapeutique transdermique, le système d'application thérapeutique transdermique étant un pansement ou un patch comprenant une couche matricielle contenant une substance active et une couche dorsale imperméable.

9. Composé ou composition à utiliser selon la revendication 8, le système d'application thérapeutique transdermique étant un réservoir de substance active, qui a un fond perméable faisant face à la peau du sujet.
